# EUROPEAN PATENT APPLICATION

(11) **EP 4 768 023 A1**
(43) Date of publication of application: **01.07.2026**
(21) Application number: 25209958.5
(22) Date of filing: 21.10.2025
(51) Int. Cl.: A61K 9/48, A61K 31/165

(54) **CAPSULE FORMULATIONS OF LISDEXAMFETAMINE DIMESYLATE**

(30) Priority: 24.12.2024 TR 2024020287
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); BENGUL, Furkan, Istanbul (TR)
(74) Representative: Genç Ilhan, Oznur

(57) **Abstract**

The present invention relates to a capsule formulation comprising lisdexamfetamine dimesylate and at least one stabilizer. Furthermore, the invention discloses a process which is simple, rapid, cost effective, time-saving and industrially convenient process and is created to eliminate the disadvantages of active ingredient.

## Description

### Field of the Invention

The present invention relates to a capsule formulation comprising lisdexamfetamine dimesylate and at least one stabilizer. Furthermore, the invention discloses a process which is simple, rapid, cost effective, time-saving and industrially convenient process and is created to eliminate the disadvantages of active ingredient.

### Background of the Invention

Lisdexamfetamine dimesylate is used to treat attention deficit hyperactivity disorder (ADHD) in adults and children 6 years of age and older. This medicine is also used to treat moderate to severe binge eating disorder (BED). It belongs to the group of medicines called central nervous system (CNS) stimulants.

Lisdexamfetamine dimesylate increases attention and decreases restlessness in children and adults who are overactive, cannot concentrate for very long, or are easily distracted and impulsive. This medicine is used as part of a total treatment program that also includes social, educational, and psychological treatment.

Lisdexamfetamine dimesylate also referred to as I-lysine-d-amphetamine dimesylate of Formula I is chemically named as (2S)-2,6-Diamino-N-[(1S)-1-methyl-2-phenylethyl]-hexanamide dimesylate.

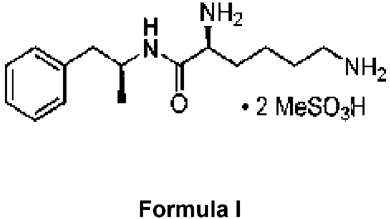

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is currently commercially available only as 10, 20, 30, 40, 50 and 60 mg hard capsules or chewable tablets. Each capsule or chewable tablet contains 10, 20, 30, 40, 50 and 60 mg lisdexamfetamine dimesylate, equivalent to 5.8 mg, 11.6 mg, 17.3 mg, 23.1 mg or 28.9 mg lisdexamfetamine, respectively.

The patent WO2006121552A2 discloses a capsule formulation containing lisdexamfetamine dimesylate, microcrystalline cellulose, croscarmellose sodium, magnesium stearate.

The formulation comprising the capsule form of lisdexamfetamine dimesylate is available in the prior art. However, there is still a need for a high stability formulation to be developed taking into account the disadvantages of lisdexamfetamine dimesylate. Lisdexamfetamine dimesylate is an active substance that is deliquescent and needs to be formulated accordingly. By using lisdexamfetamine dimesylate with a stabilizer, we have obtained a formulation that reduces moisture absorption and thus has high stability.

### Detailed Description of the Invention

The main object of the present invention is to provide a capsule formulation comprising lisdexamfetamine dimesylate with high stability.

Another object of the present invention is to provide a capsule formulation comprising lisdexamfetamine dimesylate having the desired dissolution profile and content uniformity.

Another object of the present invention is to provide a method for a capsule formulation comprising lisdexamfetamine dimesylate prepared by simple, easy, time-saving and fast manufacturing methods.

High humidity in the environment may affect the uniformity of dosage form and stability of the product. Therefore, it is important that lisdexamfetamine dimesylate is protected from moisture to maintain its stability. We aimed to reduce the moisture absorption capacity of the dosage form and increase the stability of lisdexamfetamine dimesylate by using stabilizers in the formulation. Surprisingly, we found that moisture absorption was reduced when stabilizer was used, thus obtaining a formulation with high stability.

According to one embodiment of the invention, a capsule formulation comprising lisdexamfetamine dimesylate and at least one stabilizer.

According to one embodiment of the present invention, the amount of the lisdexamfetamine dimesylate is between 30.0% and 80.0% by weight of the total formulation. The amount of the lisdexamfetamine dimesylate is between 30.0% and 50.0%, preferably, between 34.0% and 46.0% by weight of the total formulation.

Suitable stabilizers are selected from the group comprising magnesium aluminometasilicate, glycerol dibehenate, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

According to one embodiment of the present invention, the stabilizer is magnesium aluminometasilicate and glycerol dibehenate or mixture thereof. When these were used, thus obtaining a formulation with high stability.

According to one embodiment of the present invention, the stabilizer is magnesium aluminometasilicate.

According to one embodiment of the present invention, the stabilizer is glycerol dibehenate. It is also known that glyceryl dibehenate is used as a moisture protective agent.

According to one embodiment of the present invention, the amount of the stabilizer is between 1.0% and 20.0% by weight of the total formulation. Preferably, it is between 3.0% and 12.0% by weight in the total formulation.

According to one embodiment of the present invention, the capsule formulation further comprises at least one pharmaceutically acceptable excipient selected from the group comprising fillers, disintegrants, binders, lubricants or mixtures thereof.

Suitable fillers are selected from the group comprising microcrystalline cellulose, lactose, starch, sucrose, ammonium alginate, calcium phosphate, dibasic calcium phosphate, neutral pellets, calcium sulfate, cellulose acetate, erythritol, ethyl cellulose, fructose, glyceryl palmitostearate, mannitol, magnesium carbonate, magnesium oxide, maltodextrin, maltose, medium chain triglycerides, polydextrose, polymethacrylates, sodium chloride, sorbitol, sugar spheres, tragacanth, trehalose, polysorbate 80, xylitol or mixtures thereof.

According to this embodiment of the present invention, the filler is microcrystalline cellulose.

In addition, the moisture content of microcrystalline cellulose was used below 1.5%. In this way, it helped to increase the stability of lisdexamfetamine dimesylate, which is sensitive to moisture.

According to this embodiment of the present invention, the amount of filler is between 35.0% and 60.0% by weight of the total formulation, preferably it is between 40.0% and 55.0% by weight of the total formulation.

Suitable disintegrants are selected from the group comprising croscarmellose sodium, sodium starch glycolate, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

According to one embodiment of the present invention, the disintegrant is croscarmellose sodium.

According to one embodiment of the present invention, the amount of disintegrant is between 0.1% and 10.0% by weight of the total formulation, preferably it is between 2.0% and 6.0% by weight of the total formulation.

Suitable binder in the mixture are selected from the group comprising sodium carboxymethyl cellulose, polyvinylpyrrolidone (K30, K90), sodium carboxymethyl cellulose, polyethylene glycol, starch, pregelatinized starch, sodium alginate, hydroxypropyl methyl cellulose, hydroxypropyl cellulose, carboxy methyl cellulose, methyl cellulose, gelatin, carrageenan, guar gum, polymethacrylates, methacrylate polymers, hyaluronic acid, pectin, polysaccharides, carbomer, poloxamer, polyacrylamide, aluminium hydroxide, polyoxyethylene-alkyl ether, polydextrose, polyethylene oxide or mixtures thereof.

According to one embodiment of the present invention, the amount of binder is between 0.5% and 10.0% by weight in the total formulation.

Suitable lubricants are selected from the group comprising sodium stearyl fumarate, magnesium stearate, talc, colloidal silicon dioxide, corn, calcium stearate, zinc stearate, magnesium lauryl sulfate, sodium oleate, sodium acetate, stearic acid, fumaric acid, or mixtures thereof.

According to one embodiment of the present invention, the lubricant is sodium stearyl fumarate. The selected lubricant ensures uniformity of content and thus the desired dissolution profile.

According to one embodiment of the present invention, the amount of lubricant is between 0.1% and 5.0% by weight in the total formulation.

According to one embodiment of the present invention, a capsule formulation comprises;
- Lisdexamfetamine dimesylate
- Microcrystalline cellulose
- Magnesium aluminometasilicate
- Croscarmellose sodium
- Sodium stearyl fumarate

According to one embodiment of the present invention, a capsule formulation comprises;
- Lisdexamfetamine dimesylate
- Microcrystalline cellulose
- Glycerol dibehenate
- Croscarmellose sodium

According to this embodiment of the invention, a process for capsule formulations comprises the following steps:
a. Sieving and blending Lisdexamfetamine Dimesylate and stabilizer,
b. Sieving filler and disintegrant, and blending them with the powder mixture obtained in (a),
c. Sieving the lubricant and blending it with the powder mixture obtained in (b),
d. Filling the final mixture obtained in (c) into capsules.

According to this embodiment of the invention, a process for capsule formulations comprises the following steps:
a. Sieving and blending Lisdexamfetamine Dimesylate and stabilizer,
b. Sieving filler and disintegrant, and blending them with the powder mixture obtained in (a),
c. Filling the final mixture obtained in (b) into capsules.

### Example 1;

| **Ingredient** | **%by weight** | **%by weight** |
|---|---|---|
| Lisdexamfetamine Dimesylate | 40 | 30-50 |
| Microcrystalline Cellulose | 45.7 | 35-60 |
| Magnesium Aluminometasilicate | 9.7 | 1-20 |
| Croscarmellose Sodium | 2.9 | 0.1-10 |
| Sodium stearyl fumarate | 1.7 | 0.1-5 |
| **TOTAL** | **100** | **100** |
| **Gelatin capsule** | | |

a. Sieving and blending Lisdexamfetamine Dimesylate and Magnesium Aluminometasilicate,
b. Sieving Microcrystalline Cellulose and Croscarmellose Sodium and blending them with the powder mixture obtained in (a),
c. Sieving the sodium stearyl fumarate and blending it with the powder mixture obtained in (b),
d. Filling the final mixture obtained in (c) into capsules.

### Example 2;

| **Ingridient** | **%by weight** | **%by weight** |
|---|---|---|
| Lisdexamfetamine Dimesylate | 38.9 | 30-50 |
| Microcrystalline Cellulose | 51.7 | 40-60 |
| Glycerol Dibehenate | 6.7 | 1-20 |
| Croscarmellose Sodium | 2.7 | 0.1-10 |
| **TOTAL** | **100** | **100** |
| **Gelatin capsule** | | |

a. Sieving and blending Lisdexamfetamine Dimesylate and Glycerol dibehenate,
b. Sieving Microcrystalline Cellulose and Croscarmellose Sodium and blending them with the powder mixture obtained in (a),
c. Filling the final mixture obtained in (b) into capsules.

## Claims

1. A capsule formulation comprising lisdexamfetamine dimesylate and at least one stabilizer.

2. The capsule formulation according to claim 1, wherein the amount of the lisdexamfetamine dimesylate is between 30.0% and 80.0% by weight of the total formulation.

3. The capsule formulation according to claim 1, wherein stabilizers are selected from the group comprising magnesium aluminometasilicate, glycerol dibehenate, citric acid, fumaric acid, tartaric acid, sodium citrate, sodium benzoate, sodium dihydrogen phosphate, calcium carbonate, magnesium carbonate, arginine, lysine, meglumine, ascorbic acid, gallic acid esters or the mixtures thereof.

4. The capsule formulation according to claim 1 or 3, wherein the stabilizer is magnesium aluminometasilicate and glycerol dibehenate or mixture thereof.

5. The capsule formulation according to claim 3 or 4, wherein the amount of the stabilizer is between 1.0% and 20.0% by weight of the total formulation.

6. The capsule formulation according to claim 1, wherein the capsule formulation further comprises at least one pharmaceutically acceptable excipient selected from the group comprising fillers, disintegrants, lubricants or mixtures thereof.

7. The capsule formulation according to claim 6, wherein fillers are selected from the group comprising microcrystalline cellulose, lactose, starch, sucrose, ammonium alginate, calcium phosphate, dibasic calcium phosphate, neutral pellets, calcium sulfate, cellulose acetate, erythritol, ethyl cellulose, fructose, glyceryl palmitostearate, mannitol, magnesium carbonate, magnesium oxide, maltodextrin, maltose, medium chain triglycerides, , polydextrose, polymethacrylates, sodium chloride, sorbitol, sugar spheres, tragacanth, trehalose, polysorbate 80, xylitol or mixtures thereof.

8. The capsule formulation according to claim 7, wherein the amount of filler is between 35.0% and 60.0% by weight of the total formulation.

9. The capsule formulation according to claim 6, wherein disintegrants are selected from the group comprising croscarmellose sodium, sodium starch glycolate, crospovidone, low-substituted hydroxypropyl cellulose, starch, calcium carboxymethyl cellulose, pregelatinized starch, docusate sodium, guar gum, sodium alginate, alginic acid, magnesium aluminum silica, poloxamer, sodium glycine carbonate or mixtures thereof.

10. The capsule formulation according to claim 9, wherein the amount of disintegrant is between 0.1% and 5.0% by weight of the total formulation.

11. The capsule formulation according to claim 6, wherein lubricants are selected from the group comprising sodium stearyl fumarate, magnesium stearate, talc, colloidal silicon dioxide, corn, calcium stearate, zinc stearate, magnesium lauryl sulfate, sodium oleate, sodium acetate, stearic acid, fumaric acid, or mixtures thereof.

12. The capsule formulation according to claim 11, wherein the lubricant is sodium stearyl fumarate.

13. The capsule formulation according to claim 11 or 12, wherein the amount of lubricant is between 0.1% and 5.0% by weight in the total formulation.

14. A process for capsule formulation according to any of the preceding claims comprising lisdexamfetamine dimesylate comprises the following steps:
a. Sieving and blending lisdexamfetamine dimesylate and stabilizer,
b. Sieving filler and disintegrant, and blending them with the powder mixture obtained in (a),
c. Filling the final mixture obtained in (b) into capsules.
